# EUROPEAN PATENT SPECIFICATION

(11) **EP 2 635 237 B1**
(45) Date of publication and mention of the grant of the patent: **25.02.2015**
(21) Application number: 11755150.7
(22) Date of filing: 24.08.2011
(51) Int. Cl.: A61F 2/38

(54) **TOTAL KNEE PROSTHESIS**
TOTALKNIEPROTHESE
PROTHÈSE TOTALE DU GENOU

(30) Priority: 04.11.2010 FR 1059103
(43) Date of publication of application: 11.09.2013
(73) Proprietor: Kokab, 5413 Canach (LU)
(72) Inventor: Landanger, Joël, 52330 Colombey Les Deux Eglises (FR)
(74) Representative: Jeannet, Olivier
(86) International application number: PCT/IB2011/053713
(87) International publication number: WO 2012/059825

(56) References cited:
- EP-A1- 1 354 571
- WO-A1-2010/001010
- DE-U1-202010 000 037
- FR-A1- 2 796 836
- FR-A1- 2 852 819
- US-A1- 2009 204 221

## Description

The present invention relates to a total knee prosthesis.

A total knee prosthesis, as this is well known, comprises a femoral implant reproducing the femoral condyles and a tibial implant reproducing the tibial plateau, which comprises glenoid surfaces for receiving these condyles. This tibial implant may comprise a base part intended to be attached to the tibia and a sliding element in a material promoting sliding, notably in high density polyethylene, mounted on this base.

Patent application publications nos. WO 2010/001010 A1, which forms the basis for the preamble of claim 1, and further US 2009/204221 A1, FR 2 852 819 A1 , EP 1 354 571 A1, FR2796836A1 and DE 20 2010 000037 U1 illustrate various prostheses of the prior art.

In the case of ablation of two crossed ligaments, implantation of a so-called "postero-stabilized" prosthesis is indicated, wherein the femoral implant comprises a rounded boss between both condyles and the tibial implant comprises a median protuberance; during the flexural movement, said boss will encounter said protuberance, which allows suppression of the risk of anterior luxation of the tibia relatively to the femur.

This type of "postero-stabilized" prosthesis however has significant drawbacks in that it generates more or less pronounced impacts of said boss against said protuberance, which severely put a strain on the bone anchorings of the tibial and femoral implants with the risk of causing their being freed in the long run, of undergoing notable wear at said protuberance.

The present invention aims at finding a remedy to these essential drawbacks.

The relevant prosthesis in a way known *per se* comprises a femoral implant forming two condylian portions which reproduce the femoral condyles, and a tibial implant comprising an intermediate plate forming glenoid surfaces for receiving the condylian portions and comprising a median protuberance.

According to the invention,
- said condylian portions have a curved shape both in the sagittal plane and in the transverse plane and the intermediate plate has raised anterior, lateral and posterior edges at the periphery of the glenoid surfaces, said condylian portions and the glenoid surfaces being congruent, i.e. fitting together two by two over the whole of the movement of the joint or over the major portion of this movement;
- the femoral implant comprises an anterior inter-condylian wall forming a lower inter-condylian edge; and
- said protuberance is elongated in the antero-posterior direction, and extends over the whole of the antero-posterior dimension of the intermediate plate; it has a posterior boss with a convex upper face in the sagittal plane, which plunges down towards the central area of the protuberance, and an anterior portion with a concave upper face in the sagittal plane which emerges from this same central area, said central area thus having a recess continuously connected to the posterior boss, while forming a smooth transition with the upper face of the latter; this recess is located so that in an extension or hyperextension of the joint, said lower inter-condylian edge is immediately below it, or slightly engaged on the anterior portion of the posterior boss.

Thus, the prosthesis according to the invention has, as a combination, condylian portions and strongly congruent glenoid surfaces, a lower inter-condylian edge and a median protuberance forming a central recessed area having a smooth transition with a posterior boss with a convex upper face. This strong congruence and the fact that the protuberance extends over the whole of the intermediate plate in the antero-posterior direction give the possibility of obtaining significant stability of the prosthesis over the whole of its joint movement, even in the case of ablation of the crossed ligaments.

Said concave anterior portion of the protuberance forms an open space, which allows said anterior inter-condylian wall to become engaged above it when the femoral implant heads for its position corresponding to the extension of the joint; in the extension or hyperextension position of this joint, the inter-condylian edge comes into soft contact with the anterior portion of the posterior boss, eliminating any problem of impact of the femoral implant against the tibial implant during this movement.

The prosthesis according to the invention thus puts a weak strain on the bone anchorings of the femoral and tibial implants, and, additionally, is subject to low wear at said protuberance.

Preferably, said central area is also continuously connected to said anterior portion of the protuberance, while forming a smooth transition with the upper face of the latter.

Advantageously, said protuberance has two longitudinal flanks forming marked angles with the adjacent glenoid surfaces and rounded angles with the upper faces of the posterior boss and of said anterior portion.

Reinforced guidance of the femoral implant relatively to the tibial implant is thus obtained by means of said marked angles, without any substantial wear being generated at the angles formed by these natural flanks and by the upper faces of the posterior boss and of said anterior portion in the case of play of the femoral implant relatively to the tibial implant in the transverse direction, by means of said rounded angles.

Preferably, the upper wall of the posterior boss is convex in the transverse plane, for example, along a curvature substantially identical with the one which this boss has in the sagittal plane.

The posterior boss thus provides a smooth transition between its upper face and said rounded edges of the angles between this upper face and the flanks of the protuberance, reducing the risk of wear of the boss at the posterior portion of the latter.

Preferably, said lower inter-condylian edge has a curved shape, and said posterior boss has rounded anterior lateral portions, giving the anterior portion of this boss an anterior shape which is also curved.

These curved shapes of the inter-condylian edge, and of the anterior portion of the posterior boss allow an increase in the contact surface area of this inter-condylian edge with the posterior boss, therefore, a distribution of the stresses exerted on the latter, and accordingly a reduction of the wear to which it is subject. Preferably, the tibial implant comprises:
- a base for attachment to the bone of the tibia, forming an upper planar wall and a cylindrical and/or conical well opening out into this planar wall, and
- a sliding element in a material promoting sliding of the femoral implant, notably in high density polyethylene, including said intermediate plate and a pin, which may be pivotally received in said well.

The sliding element is thus pivotally mobile, relatively to the base for attachment to the bone.

The invention will be better understood and other characteristics and advantages thereof will become apparent with reference to the appended schematic drawing illustrating as a non-limiting example a preferred embodiment of the total knee prosthesis to which it relates.
Fig. 1 is an exploded perspective view their all, at a first angle;
Fig. 2 is a similar view thereof, at another angle;
Fig. 3 is a view thereof similar to Fig. 2, after assembling the constitutive elements of the prosthesis;
Fig. 4 is a posterior side view of an intermediate sliding element which comprises;
Fig. 5 is a view of this sliding element, from above;
Fig. 6 is a sectional view of the sliding element along the line VI-VI of Fig. 4;
Fig. 7 is a sectional view of the sliding element along the line VII-VII of Fig. 4, and
Fig. 8 is a sectional view thereof along the line VIII-VIII de la figure 5.

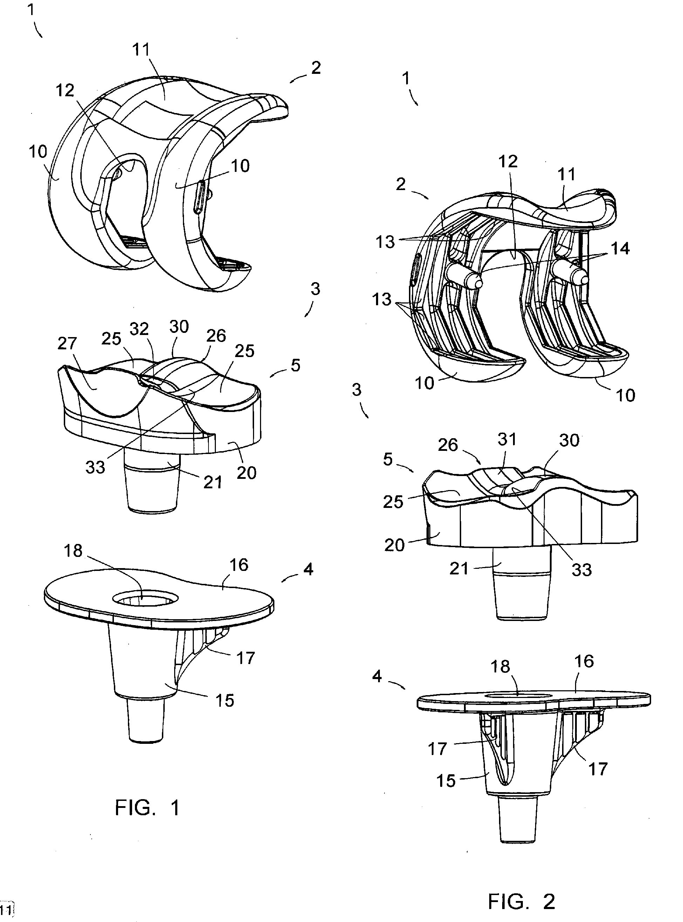

Figs. 1 to 3 represent a total knee prosthesis 1, comprising a femoral implant 2 and a tibial implant 3. The latter is formed with a base 4 intended to be attached to the tibial and with an intermediate sliding element 5.

The femoral implant 2 and the base 4 are in metal material, whereas the element 5 is in a material promoting sliding, notably high-density polyethylene.

The femoral implant 2 has a curved shape, substantially "as a shield" capable of surrounding the lower end of a duly resected femur. It comprises two curved lateral portions 10 and an anterior wall 11, which, on the external side of the implant 2, reproduce the femoral condyles and the trochlea of a femur, respectively. At its lower end, the wall 11 forms an inter-condylian edge 12 with a curved shape.

The radius of curvature of the portion of each lateral portion 10 extending beyond the wall 11 is of the order of 30 mm in the sagittal plane and also of the order of 30 mm in the transverse plane. The radius of curvature of the edge 12 is of the order of 9 to 10 mm.

On its internal side, as shown in Fig. 2, the implant 2 has anterior, distal, posterior and intermediate supporting walls 13, bearing against the resected bone, and two pads 14 intended to be inserted into the spongy bone of the femur.

The base 4 comprises a lower pad 15 intended to be inserted into the spongy bone of the tibia, an upper planar wall 16 intended to rest against a proximal resected face of the tibia, and stabilization fins 17 in the bone, connecting this pad and this plate. The pad 15 is hollow and forms a cylindrical and/or conical well 18 opening into the upper face of the wall 16.

The sliding element 5 has an upper portion forming an intermediate plate 20, the lower face of which is planar and is intended to rest against the wall 16, and a cylindrical and conical lower pin 21 at its lower end, intended to be pivotally received in the well 18.

The intermediate plate 20 forms glenoid surfaces 25 for receiving condylian portions 10 of the femoral implant 2, and comprises a median protuberance 26. It further has an anatomic anterior recess 27 for letting through the patellar ligament.

It has raised anterior, lateral and posterior edges at the periphery of the glenoid surfaces 25, such that the condylian portions 10 and these glenoid surfaces 25 are congruent, i.e. fitting together two by two on the whole of the movement of the joint, or on the major part of this movement.

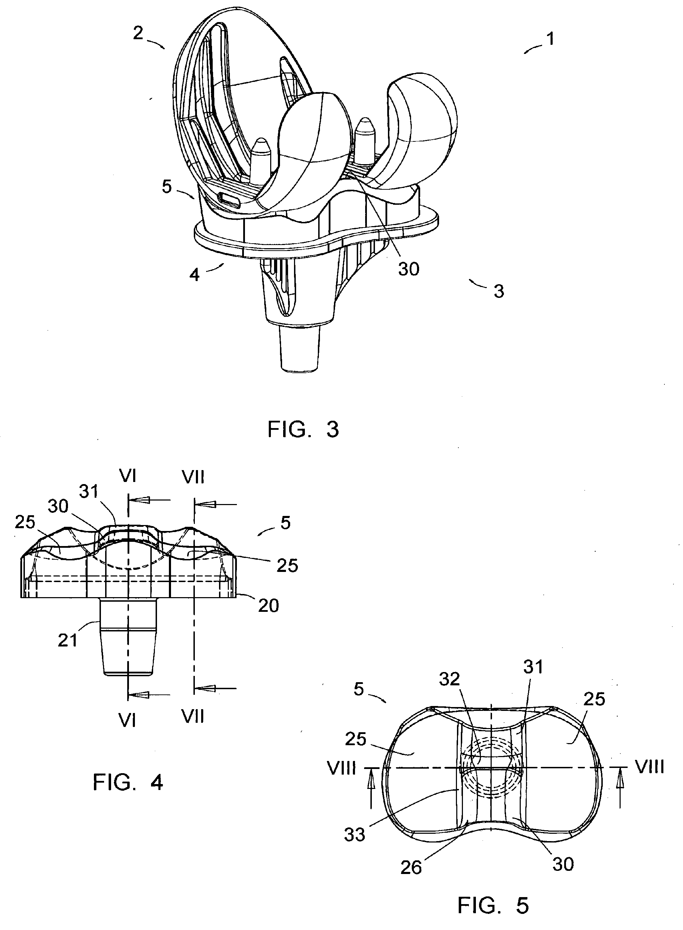

As more particularly shown in Figs. 4 to 8, the protuberance 26 is elongated in the antero-posterior direction, and extends over the whole of the antero-posterior dimension of the intermediate plate 20. It has a posterior boss 30 and an anterior portion 31 defining between them a more or less central area 32 of the protuberance, as well as longitudinal flanks 33.

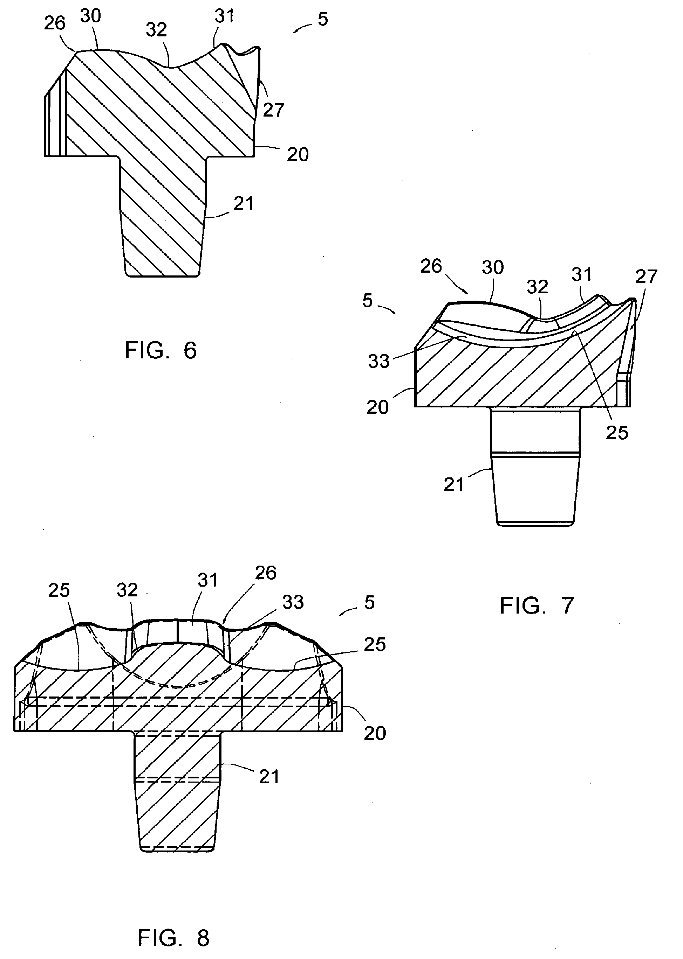

The posterior boss 30 has a convex upper face in the sagittal plane (Figs. 6 and 7), and plunges in the direction of the central area 32 of the protuberance, i.e. it tilts downwards from its posterior portion to the anterior portion. At its junction with this central area 32, it has rounded anterior lateral portions, giving its anterior portion a shape having a curvature close to that of the inter-condylian edge 12.

The upper face of the boss 30 is also convex in the transverse plane (Fig. 8), thus providing a smooth transition between its upper face and its connecting angles to the flanks 33 of the protuberance, these angles being rounded.

The anterior portion 31 of the protuberance 26 has a slightly concave upper face in the sagittal plane (Figs. 6 and 7), and which emerges from said central area 32, i.e., which extends upwards from its posterior portion to its anterior portion. The tilt of this portion 31 substantially follows the raising of the anterior edge of the intermediate plate 20.

Said anterior portion 31 is, like the boss 30, connected to the flanks 33 through rounded angles.

As this is particularly apparent in Figs. 6 and 7, the area 32 is continuously connected to the posterior boss 30 and to said anterior portion 31, and has a smooth transition with the upper faces of this boss 30 and of this anterior portion 31. The recess which this area 32 forms between the latter is located so that in the extension or hyperextension position, the inter-condylian edge 12 is particularly below it, or slightly engaged onto the anterior portion of the boss 30.

The longitudinal flanks 33 of the protuberance 26 extend over the whole of the width of the intermediate plate 20. They form marked angles with the adjacent glenoid surfaces 25 and the aforementioned rounded angles with the upper faces of the boss 30 and of said anterior portion 31.

As this is understood, the prosthesis 1 has, as a combination, strongly congruent condylian portions 10 and glenoid surfaces 25, an inter-condylian edge 12 and a median protuberance 26 forming a central recessed area 32, having a smooth transition with a posterior boss 30 with a convex upper face. This strong congruence and the fact that the protuberance 26 extends over the whole of the intermediate plate 20 in the antero-posterior direction gives the possibility of obtaining significant stability of the prosthesis 1 on the whole of its joint movement, even in the case of ablation of the crossed ligaments.

Said anterior portion 31 forms an open space with which the inter-condylian wall 11 may engage above it when the femoral implant 2 makes its way towards its position corresponding to the extension of the joint; in the extension or hyperextension position of this joint, the inter-condylian edge 12 comes into soft contact with the anterior portion of the boss 30, suppressing any problem of impact of the femoral implant 2 against the tibial implant 3 during this movement.

The flanks 33 as for them allow reinforced guiding of the femoral implant 2 relatively to the tibial implant 3 by said marked angles without there being generated any substantial wear at the angles formed by these flanks 33, and by the upper faces of the boss 30 and of said anterior portion 31 in the case of play of the femoral implant 2 relatively to the tibial implant 3 in the transverse direction, by said rounded angles.

As is apparent from the foregoing, the invention provides a total knee prosthesis having, as compared with the homologous prostheses of the prior art, determining advantages of only putting a weak strain on the bone anchorings of the femoral and tibial implants and of being subject to low wear at said protuberance 32.

The invention has been described above, with reference to an embodiment, given as an example; it is obvious that it is not limited to this embodiment, but on the contrary, it encompasses all the alternative embodiments covered by the appended claims.

## Claims

1. A total knee prosthesis (1) comprising a femoral implant (2) forming two condylian portions which reproduce the femoral condyles, and a tibial implant (3) comprising an intermediate plate (20) forming glenoid surfaces (25) for receiving the condylian portions (10) and comprising a median protuberance (26);
- said condylian portions (10) have a curved shape both in the sagittal plane and in the transverse plane and the intermediate plate (20) has raised anterior, lateral and posterior edges at the periphery of the glenoid surfaces (25), said condylian portions (10) and the glenoid surfaces (25) being congruent, i.e. fitting together two by two over the whole of the movement of the joint or over the major portion of this movement;
- said protuberance (26) is elongated in the antero-posterior direction, and extends over the whole of the antero-posterior dimension of the intermediate plate (20); it has a posterior boss (30) with a convex upper face in the sagittal plane, which plunges down towards the central area (32) of the protuberance (26), and an anterior portion (31) with a concave upper face in the sagittal plane which emerges from this same central area (32), said central area (32) thus having a recess continuously connected to the posterior boss (30), while forming a smooth transition with the upper face of the latter;
**characterized in that**:
- the femoral implant (2) comprises an anterior inter-condylian wall (11) forming a lower inter-condylian edge (12); and
- said recess is located so that in an extension or hyperextension of the joint, said lower inter-condylian edge (12) is immediately below it, or slightly engaged on the anterior portion of the posterior boss (30).

2. The prosthesis according to claim 1, wherein said central area (32) is also continuously connected to said anterior portion (31) of the protuberance (26), while forming a smooth transition with the upper face of the latter.

3. The prosthesis accordi n g to claim 1 or claim 2, wherein said protuberance (26) has two longitudinal flanks (33) forming marked angles with the adjacent glenoid surfaces (25) and rounded angles with the upper faces of the posterior boss (30) and of said anterior portion (31).

4. The prosthesis according to anyone of claims 1 to 3, wherein the upper wall of the posterior boss (30) is convex in the transverse plane.

5. The prosthesis according to anyone of claims 1 to 4, wherein said lower inter-condylian edge (12) has a curved shape, and said posterior boss (30) has rounded anterior lateral portions, giving the anterior portion of this boss an anterior shape which is also curved.

6. The prosthesis according to claim 5, wherein the radius of curvature of lower inter-condylian edge (12) is of the order of 9 to 10 mm.

7. The prosthesis according to anyone of claims 1 to 6, wherein the tibial implant (3) comprises:
- a base (4) for attachment to the bone of the tibia, forming an upper planar wall (16) and a cylindrical and/or conical well (18) opening out into this planar wall (16), and
- a sliding element (5) in a material promoting sliding of the femoral implant, notably in high density polyethylene, including said intermediate plate (20) and a pin (21), which may be pivotally received in said well (18).

8. The prosthesis according to anyone of claims 1 to 7, wherein the radius of curvature of condylian portions (10) extending from the inter-condylian wall (11) is of the order of 30 mm in the sagittal plane, and also of the order of 30 mm in the transverse plane.

## Patentansprüche

1. Eine Kniegelenksprothese (1) mit einem Oberschenkelimplantat (2) bildenden beiden kondylären Abschnitte (10), die die Femurkondylen reproduziert, und ein Schienbeinimplantat (3) eine Platte (20) bildet glenoidalen Oberfläche (25) Empfangen der kondylären Abschnitte (10) und umfassend einen Eminentia mediana (26);
- wobei kondylären Abschnitte (10) eine gekrümmte Form haben sowohl in der Sagittalebene und in der Querebene und Tibiaplateaus (20) eine Vorderkante, Seitenkanten und einer hinteren Kante, die an der Peripherie der glenoidalen Oberfläche (25) angehoben werden, wobei die kondylären Abschnitte (10) und der glenoidalen Oberfläche (25) kongruent sind, das heißt, sie sind paarweise auf der gesamten Bewegung der Verbindungsstelle oder auf den Hauptteil dieser Bewegung zusammengebaut,
- wobei der Eminentia (26) in der Antero-Posterioren Richtung länglich ist und erstreckt sich über die gesamte Anterior-Posterior-Abmessung des Tibiaplateaus (20); sie hat einen hinteren Vorsprung (30) mit einer konvexen Oberfläche in der Sagittalebene die in Richtung des zentralen Bereichs (32) der Eminentia (26) eintaucht, und ein vorderer Abschnitt (31) mit einer konkaven oberen Oberfläche in der Sagittalebene, die aus dem gleichen zentralen Bereichs (32) entsteht, wobei der zentrale Bereich (32) so mit einer Aussparung kontinuierlich bis zum hinteren Vorsprung (30) verbunden ist, unter Ausbildung einer glatten Übergang mit der oberen Fläche der letzteren;
**dadurch gekennzeichnet, dass** :
- das Oberschenkelimplantat (2) eine vordere interkondylären Wand (11) beinhaltet, die eine niedrigere interkondylären Kante (12) bildet; und
- wobei die Vertiefung ist so angeordnet, dass in der ausgefahrenen Position oder in der Hyperextension Position des Gelenks, die untere Inter-condylian Kante (12) unmittelbar darunter ist oder leicht über den vorderen Teil hinteren Vorsprung (30).

2. Prothese nach Anspruch 1, wobei der zentrale Bereich (32) kontinuierlich, das mit dem vorderen Abschnitt (31) des Vorsprungs (26) unter Bildung einer glatten Übergang mit der oberen Fläche des letzteren.

3. Prothese nach Anspruch 1 oder Anspruch 2, wobei der Vorsprung (26) zwei Längsflanken (33) bildenden markierten Winkel mit den benachbarten glenoidalen Oberfläche (25) und abgerundeten Winkel mit den oberen Oberflächen der hinteren Vorsprung (30) und des der vordere Teil (31).

4. Prothese nach einem der Ansprüche 1 bis 3, wobei die obere Wand des hinteren Vorsprungs (30) konvex in der Querebene.

5. Die Prothese nach einem der Ansprüche 1 bis 4, wobei die unteren interkondylären Kante (12) eine gekrümmte Form hat, und die hinteren Vorsprungs (30) hat vorderen seitlichen Abschnitte abgerundet, so dass der vordere Teil dieser Vorsprung eine vordere Form, die auch gekrümmt.

6. Prothese (1) nach Anspruch 5, wobei der Krümmungsradius des unteren interkondylären Kante (12), in der Größenordnung von 9 bis 10 mm ist.

7. Prothese nach einem der Ansprüche 1 bis 6, wobei das Schienbeinimplantat (3) umfasst:
- eine Basis (4) zur Befestigung an dem Tibiaknochen, Bilden einer ebenen oberen Wand (16) und einen zylindrischen Schaft und / oder konische (18) Öffnung in der ebenen Wand (16), und
- Ein Schiebeelement (5) in einem Material zu fördern Gleiten des femoralen Implantats, insbesondere in Polyethylen hoher Dichte, einschließlich der Zwischenplatte (20) und einen Stift (21), die schwenkbar in die empfangen werden können dem Schacht (18).

8. Prothese nach einem der Ansprüche 1 bis 7, wobei der Krümmungsradius der kondylären Abschnitte (10) sich von der interkondylären Wand (11) ist in der Größenordnung von 30 mm in der Sagittalebene, als auch in der Größenordnung von 30 mm in der Querebene.

## Revendications

1. Une prothèse totale du genou (1), comprenant un implant fémoral (2) formant deux parties condyliennes (10) qui reproduisent les condyles fémoraux, et un implant tibial (3) comprenant un plateau (20) formant des surfaces glénoïdes (25) de réception desdites parties condyliennes (10) et comprenant une éminence médiane (26) ;
- lesdites parties condyliennes (10) présentent une forme courbe tant dans le plan sagittal que dans le plan transverse et le plateau tibial (20) présente des bords antérieur, latéraux et postérieur relevés au niveau de la périphérie des surfaces glénoïdes (25), lesdites parties condyliennes (10) et surfaces glénoïdes (25) étant congruentes, c'est-à-dire s'épousant deux à deux sur l'ensemble du mouvement de l'articulation, ou sur la majeure partie de ce mouvement ;
- ladite éminence (26) est allongée dans la direction antéro-postérieure et s'étend sur l'ensemble de la dimension antéro-postérieure du plateau tibial (20) ; elle présente un bossage postérieur (30) à face supérieure convexe dans le plan sagittal, qui plonge en direction de la zone centrale (32) de l'éminence (26), et une partie antérieure (31) à face supérieure concave dans le plan sagittal, qui émerge de cette même zone centrale (32), ladite zone centrale (32) présentant ainsi un creux relié de façon continue au bossage postérieur (30), en formant une transition douce avec la face supérieure de celui-ci ;
**caractérisée en ce que** :
- l'implant fémoral (2) comprend une paroi inter-condylienne antérieure (11) formant un bord inter-condylien inférieur (12) ; et
- ledit creux est situé de telle sorte qu'en position d'extension ou d'hyperextension de l'articulation, ledit bord inter-condylien inférieur (12) soit à l'aplomb de lui ou en léger engagement sur la portion antérieure du bossage postérieur (30).

2. La prothèse (1) selon la revendication 1, dans laquelle ladite zone centrale (32) est également reliée de façon continue à ladite partie antérieure (31) de l'éminence (26), en formant une transition douce avec la face supérieure de celle-ci.

3. La prothèse (1) selon la revendication 1 ou la revendication 2, dans laquelle ladite éminence (26) présente deux flancs longitudinaux (33) formant des angles marqués avec les surfaces glénoïdes (25) adjacentes et des angles arrondis avec les faces supérieures du bossage postérieur (30) et de ladite partie antérieure (31).

4. La prothèse (1) selon l'une des revendications 1 à 3, dans laquelle la paroi supérieure du bossage postérieur (30) est convexe dans le plan transverse.

5. La prothèse (1) selon l'une des revendications 1 à 4, dans laquelle ledit bord inter-condylien inférieur (12) présente une forme courbe, et ledit bossage postérieur (30) présente des parties latérales antérieures arrondies, donnant à la portion antérieure de ce bossage une forme antérieure également courbe.

6. La prothèse (1) selon la revendication 5, dans laquelle le rayon de courbure du bord inter-condylien inférieur (12) est de l'ordre de 9 à 10 mm.

7. La prothèse (1) selon l'une des revendications 1 à 6, dans laquelle l'implant tibial (3) comprend :
- une embase (4) de fixation à l'os du tibia, formant une paroi plane supérieure (16) et un puits cylindrique et/ou conique (18) débouchant dans cette paroi plane (16), et
- un élément de glissement (5) en un matériau favorisant le glissement de l'implant fémoral, notamment en polyéthylène à haute densité, incluant ledit plateau (20) et un pion (21) apte à être reçu à pivotement dans ledit puits (18).

8. La prothèse (1) selon l'une des revendications 1 à 7, **caractérisée en ce que** le rayon de courbure de portions condyliennes (10) s'étendant depuis la paroi inter-condylienne (11) est de l'ordre de 30 mm dans le plan sagittal et également de l'ordre de 30 mm dans le plan transverse.
